(19) Europäisches Patentamt
European Patent Office
Office européen des brevets

(11) **EP 2 795 241 B1**

(12) **EUROPEAN PATENT SPECIFICATION**

(45) Date of publication and mention
of the grant of the patent:
**24.02.2021 Bulletin 2021/08**

(51) Int Cl.:
*G01B 9/02* (2006.01)    *A61B 3/10* (2006.01)

(21) Application number: **12813326.1**

(22) Date of filing: **19.12.2012**

(86) International application number:
**PCT/EP2012/076130**

(87) International publication number:
**WO 2013/092697 (27.06.2013 Gazette 2013/26)**

(54) **APPARATUS AND METHOD FOR OPTICAL SWEPT-SOURCE COHERENCE TOMOGRAPHY**

VORRICHTUNG UND VERFAHREN FÜR EINE OPTISCHE
SWEPT-SOURCE-KOHÄRENZTOMOGRAFIE

APPAREIL ET PROCÉDÉ POUR TOMOGRAPHIE À COHÉRENCE DE SOURCE BALAYÉE
OPTIQUE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **21.12.2011 US 201113332727**

(43) Date of publication of application:
**29.10.2014 Bulletin 2014/44**

(73) Proprietor: **Alcon Inc.**
**1701 Fribourg (CH)**

(72) Inventors:
• **MASSOW, Ole**
  **90427 Nürnberg (DE)**
• **WISWEH, Henning**
  **91094 Langensendelbach (DE)**
• **JEGLORZ, Tobias**
  **90547 Stein (DE)**

(74) Representative: **Katérle, Axel**
**Wuesthoff & Wuesthoff**
**Patentanwälte PartG mbB**
**Schweigerstraße 2**
**81541 München (DE)**

(56) References cited:
**US-A1- 2011 176 142**

• **MICHALINA GORA ET AL.: "Ultra high-speed
swept source OCT imaging of the anterior
segment of human eye at 200 kHz with adjustable
imaging range", OPTICS EXPRESS, vol. 17, no.
17, 17 August 2009 (2009-08-17), pages
14880-14894, XP055056202, cited in the
application**
• **GEORGE Y. LIU ET AL: "High power wavelength
linearly swept mode locked fiber laser for OCT
imaging", OPTICS EXPRESS, vol. 16, no. 18, 1
September 2008 (2008-09-01), page 14095,
XP55056204, ISSN: 1094-4087, DOI:
10.1364/OE.16.014095**
• **JIEFENG XI ET AL.: "Generic real-time uniform
K-space sampling method for high-speed
swept-Source optical coherence tomography",
OPTICS EXPRESS, vol. 18, no. 9, 26 April 2010
(2010-04-26) , pages 9511-9517, XP055056201,
cited in the application**

**Description**

The present disclosure relates generally to optical coherence tomography (in short: OCT). In a preferred example, the present disclosure provides for an apparatus and a method for swept-source (in short: SS) coherence tomography that make it possible to vary the measurement depth and/or the longitudinal (axial) measurement resolution of the tomograms generated by the apparatus or by the method.

**Background of the Invention**

OCT is an imaging method based on the superposition of reference radiation and backscattered (remitted) radiation. It acquires the intensity of the interference signal, i.e. the superposed radiation fields of the reference radiation and of the remitted radiation, where the remitted radiation is backscattered at the object to be imaged. In the following, the term "light" refers to the radiation used in the case of an optical coherence tomography, which may (or may not) be outside the wavelength range that is perceptible by the human eye (visible wavelength range). In the context of the present disclosure, therefore, the reference to light is intended to include wavelengths both inside and outside the range that is visible to humans.

In the case of optical coherence tomography, a distinction can be made, in general, between OCT in the Fourier domain (in short: FD) and OCT in the time domain (in short: TD). FD-OCT, for its part, can be divided into an SD-OCT (spectral domain, in short: SD) and an SS-OCT (swept-source, in short: SS).

Prior art can be found, for example, in Optics Express 14880, vol. 17, no. 17, 17 August 2009, M. Gora *et al.,* which generally relates to "Ultra high-speed swept source OCT imaging of the anterior segment of human eye at 200 kHz with adjustable imaging range"; in US 2011/0176142 A1, which generally relates to optical coherence tomography methods and systems; in Optics Express 14095, vol. 16, no. 18, 1 September 2008, G. Y. Liu *et al.,* which generally relates to "High power wavelength linearly swept mode locked fiber laser for OCT imaging"; and in Optics Express 9511, vol. 18, no. 9, 26 April 2010, J. Xi *et al.,* which generally relates to "Generic real-time uniform K-space sampling method for high-speed swept-Source optical coherence tomography".

**Brief Summary of the Invention**

SD-OCT typically uses a light source that continuously emits broadband light of a particular spectral bandwidth $\Delta\lambda$, as well as a detector such as a spectrometer. The spectrometer in this case separates the broadband interference signal spatially into varicoloured light beams. The intensities of the individual light beams are then measured individually by a plurality of sensor elements of the detector. As a result, an intensity distribution over the wavelength $\lambda$ or over the wave number k (such as circular wave number; $k = 2\pi/\lambda$) of the detected light, an interferogram, may be obtained. This represents the interferometric unconditioned signal on the basis of which the tomogram (an A-scan) is determined. The number N of sampling points with which the interferogram is sampled may correspond to the number of sensor elements.

SS-OCT typically uses a light source that is spectrally tuneable (i.e. with respect to the wavelength of the emitted light), which instantaneously emits spectrally narrow-band light and which is tuned continuously across a spectral bandwidth $\Delta\lambda$, as well as a detector such as a single photodiode or a "balanced detector" with two photodiodes. The interferogram is acquired chronologically over the wavelength $\lambda$ or the wave number k of the detected light and sampled, with a number N of sampling points, over the course of the spectral tuning of the light source over time.

In the case of FD-OCT, the longitudinal measurement resolution $\Delta z$ (i.e. the resolution along the direction of propagation of the radiation, also referred to as "axial resolution") depends on the spectral bandwidth $\Delta\lambda$, or $\Delta k$. The following relationship can apply, in the case of Gaussian-shaped spectra, between the longitudinal measurement resolution $\Delta z$ and the spectral bandwidth:

$$\Delta z_{Gau\beta} = \frac{2\ln(2)}{n\cdot\pi}\frac{\lambda_0^2}{\Delta\lambda_{FWHM}} \tag{1}$$

It is generally applicable that:

$$\Delta k = 2\pi\frac{\Delta\lambda}{\lambda_0^2} \tag{2}$$

With (2) it follows that:

$$\Delta z_{Gau\beta} = \frac{4\ln(2)}{n}\frac{1}{\Delta k_{FWHM}} \tag{3}$$

and

$$\Delta k_{FWHM} = \frac{4\ln(2)}{n}\frac{1}{\Delta z_{Gau\beta}} \tag{4}$$

wherein n is the refractive index and $\lambda_0$ is the central wavelength of the spectrum, in this case of the Gaussian-shaped spectrum. The index 'Gauß' indicates that the corresponding quantities related to a Gaussian-shaped spectrum. The index 'FWHM' means full width at half maximum of the Gaussian-shaped spectrum. If the real part of the complex-valued interference signal is evaluated, an effective imaging depth $z_{max}$ is obtained (also called "maximally attainable measurement depth"), which is a further important parameter of FD-OCT. For the measurement depth $z_{max}$, it is possible to formulate the following dependence on the spectral bandwidth $\Delta\lambda$, or $\Delta k$, and on the number of sampling points N of the interferogram (see also Choma et al., Sensitivity advantage of swept source and Fourier domain optical coherence tomography, Optics Express, 11, 2183-2189, 2003):

$$z_{max,Gau\beta} = \frac{1}{2\cdot n}\frac{\pi}{\delta k} = \frac{1}{2\cdot n}\frac{\pi}{\Delta k_{FWHM}}N \tag{5}$$

with $\delta k$ as the wave number spacing in the case of linear sampling in k, and N as the number of samplings, or of spectral channels. From (2) and (5) one obtains:

$$z_{max,Gau\beta} = \frac{1}{4\cdot n}\frac{\lambda_0^2}{\delta\lambda} = \frac{1}{4\cdot n}\frac{\lambda_0^2}{\Delta\lambda_{FWHM}}N \tag{6}$$

wherein $\delta\lambda$ is the wavelength spacing in the case of linear sampling in $\lambda$. From (1) and (6) one obtains:

$$N = \frac{8\ln(2)}{\pi}\frac{z_{max,Gau\beta}}{\Delta z_{FWHM}} \tag{7}$$

For rectangular spectra, the following relationships apply between the longitudinal measurement resolution $\Delta z$ and the spectral bandwidth $\Delta\lambda$, or $\Delta k$:

$$\Delta z_{rec} = \frac{1}{2\cdot n}\frac{\lambda_0^2}{\Delta\lambda_{FW}} \tag{8}$$

with (2):

$$\Delta z_{rec} = \frac{\pi}{n}\frac{1}{\Delta k_{FW}} \tag{9}$$

wherein the index FW relates to the full width of the corresponding rectangular distribution. For the measurement depth $z_{max}$, the following applies:

$$z_{max,rec} = \frac{1}{2\cdot n}\frac{\pi}{\delta k} = \frac{1}{2\cdot n}\frac{\pi}{k_{FW}}N \tag{10}$$

with (2):

$$z_{max,rec} = \frac{1}{4 \cdot n} \frac{\lambda_0^2}{\delta\lambda} = \frac{1}{4 \cdot n} \frac{\lambda_0^2}{\Delta\lambda_{FW}} N \qquad (11)$$

From (8) and (11) one obtains:

$$N = 2 \frac{z_{max,rec}}{\Delta z_{FW}} \qquad (12)$$

According to the preceding formulae, the resolution $\Delta z$ and the measurement depth $z_{max}$ are determined by the parameters $\Delta\lambda$, or $\Delta k$, and N. Therefore, the resolution $\Delta z$ or/and the measurement depth $z_{max}$ can be altered by varying the spectral bandwidth $\Delta\lambda$, or $\Delta k$, or/and the number N.

SD-OCT is limited by the opto-mechanical structure of the spectrometer to a fixed, acquirable bandwidth: if the bandwidth of the light emitted by the light source is increased, the spatial divergence of the spectrum split at the dispersive element (e.g. grating or prism) of the spectrometer also increases, such that, in the case of an otherwise unaltered geometry, the original sensor elements are no longer sufficient to detect the light beams of all wavelengths/wave numbers. For a consistent number of sampling points N, therefore, either the dispersive element or the sensor should be exchanged. This is typically not possible or at least is very difficult when the OCT apparatus is operating.

More extensive information concerning optical coherence tomography and its application can be found in the following publications:

Wolfgang Drexler, James G. Fujimoto (Eds.): "Optical Coherence Tomography - Technology and Applications", Springer-Verlag Berlin Heidelberg 2008;

Jiefeng Xi et al.: "Generic real-time uniform K-space sampling method for high-speed swept-Source optical coherence tomography", Optics Express, Vol. 18, No. 9, 26 April 2010, pages 9511-9517;

Michalina Gora et al.: "Ultra high-speed swept source OCT imaging of the anterior segment of human eye at 200 kHz with adjustable imaging range", Optics Express, Vol. 17, No. 17, 17 August 2009, pages 14880-14894.

It is an object of examples as described herein to provide a user an apparatus and method for optical coherence tomography with a broad range of application in that the user can effect such variations that differing regions of an object can be imaged using, if necessary, differing longitudinal resolutions. The user may be able to switch over easily between differing measurement depths in order that, for example during an eye-surgery treatment, optionally differing sections of the treated eye can be obtained, if necessary at differing resolutions, displayed on a monitor or in another form.

The invention is set out in the independent claims. Preferred embodiments of the invention are outlined in the dependent claims.

We describe an apparatus for optical swept-source coherence tomography. The apparatus comprises a spectrally tuneable source for emitting coherent light, a detector for acquiring the intensity of interference light that results from superposing, on reference light, remitted light backscattered from an object irradiated with the coherent light of the source, and a control device. The control device is set up to control the light source and the detector in such a way that the detector performs intensity acquisitions in accordance with a defined number N of measurements, while the light source is tuned. The control device is further set up, for the purpose of altering the measurement depth $z_{max}$ and the axial resolution $\Delta z$ of the tomography, to alter a spectral measurement bandwidth $\Delta\lambda_M$ or $\Delta k_M$, within which the detector performs the intensity acquisitions.

The light source can emit coherent light of narrow, instantaneous line width, designated as $\delta_{Light\,source}\lambda$ or $\delta_{Light\,source}k$. For the purpose of performing a tomography, the light source - controlled by the control device - is tuned within a defined spectral sweep bandwidth, designated as $\Delta\lambda$ or $\Delta k$, with respect to the wavelength $\lambda$, or the wave number k, of the emitted light. The spectral sweep bandwidth $\Delta\lambda$, $\Delta k$ is delimited by the minimum wavelength $\lambda_1$ and the maximum wavelength $\lambda_2$, or the minimum wave number $k_1$ and the maximum wave number $k_2$, i.e.

$$\Delta\lambda = |\lambda_1 - \lambda_2| \qquad (13)$$

and

$$\Delta k = |k_1 - k_2| \qquad\qquad (14)$$

The centroid of the spectral sweep bandwidth $\Delta\lambda$, $\Delta k$ is $\lambda_0$ or $k_0$, respectively.

The detector - likewise controlled by the control device - can acquire the intensity of the interference light (OCT signal) at N different instants (N corresponds to the defined number of measurements). During the period of time when the N intensity measurements are performed, the light source is tuned within the measurement bandwidth $\Delta\lambda_M$ or $\Delta k_M$. In this way, a spectral interference pattern, i.e. a spectral interferogram, can be acquired. The intensity measurements performed by the detector can be processed, in a manner known per se (for instance, through use of a Fourier analysis with at least one Fourier transformation), to yield an A-scan, which represents a one-dimensional, depth-resolved reflection profile of the object to be imaged.

The intensity measurements of the detector can be performed, for example, linearly over the wavelength $\lambda$, according to a fixed timing, e.g. at regular intervals of time. For this purpose, the control device can comprise, for example, an internal timer, or the device can use an internal time signal of an AID converter connected in series to a photodiode in the detector. Alternatively, the intensity measurements can be performed linearly over the wave number k, for instance with the use of a so-called linear-k clock. Such a linear-k clock can be, for example, a fibre-based Mach-Zehnder interferometer.

The sampling intervals $\delta_{Sampling}\lambda$ or $\delta_{Sampling}k$ between the total of N successive intensity measurements can be equidistant, i.e. the measurements can be distributed at equal intervals over the wavelength $\lambda$, or over the wave number k. The spectral measurement bandwidth $\Delta\lambda_M$ or $\Delta k_M$, within which the measurements are acquired and that is significant for the measurement depth and the longitudinal resolution, is obtained, for equidistant sampling intervals, from $\Delta\lambda_M = N\cdot\delta_r\lambda$, or $\Delta k_M = N\cdot\delta_r k$. Non-equidistant sampling intervals $\delta_r\lambda$ or $\delta_r k$ are by no means precluded within the scope of the present disclosure.

In general, the value of the spectral measurement bandwidth $\Delta\lambda_M$ or $\Delta k_M$ corresponds maximally to the value of the spectral sweep bandwidth $\Delta\lambda$ or $\Delta k$ with which the light source is tuned, i.e. $\Delta\lambda_M \leq \Delta\lambda$, or $\Delta k_M \leq \Delta k$. In certain examples, the light source and the detector can be controlled, for example in a first mode, in such a way that the measurement bandwidth $\Delta\lambda_M$ or $\Delta k_M$ corresponds to the sweep bandwidth $\Delta\lambda$ or $\Delta k$. For this purpose, the N measurements (samplings) can be distributed over the entire sweep bandwidth $\Delta\lambda$ or $\Delta k$. According to the present invention, the light source and the detector are controlled in such a way that the total of N measurements (samplings) are performed only during a sub-section of the sweep bandwidth $\Delta\lambda$, $\Delta k$, and no measurements are performed outside this sub-section. In this case, the measurement bandwidth is less than the sweep bandwidth, i.e. $\Delta\lambda_M < \Delta\lambda$, or $\Delta k_M < \Delta k$.

The spectral measurement bandwidth $\Delta\lambda_M$, $\Delta k_M$ can be influenced through the number of intensity measurements and/or through the magnitude of the sampling intervals $\delta_{Sampling}\lambda$ or $\delta_{Sampling}k$.

Generally, in the case of certain examples, the measurement depth $z_{max}$ and/or the axial resolution $\Delta z$ of the tomography can be varied, in that the control device varies the measurement bandwidth $\Delta\lambda_M$, $\Delta k_M$ and/or the number N of measurements, i.e. the number of sampling points. For example, by varying the number of sampling points per spectral sweep while simultaneously keeping constant the bandwidth over which these sampling points are distributed (i.e. measurement bandwidth), the measurement depth may be varied without at the same time altering the longitudinal resolution. According to the invention, the measurement depth may be varied by altering the spectral bandwidth used during a sweep (i.e. the measurement bandwidth), but without at the same time altering the number N of measurements. In this case, the altered measurement depth involves an altered longitudinal resolution, an increased measurement depth involving a loss of resolution, and vice versa.

In order fully to exploit the performance capacity of the apparatus, an OCT apparatus according to some examples can be operated in such a way that the entire sweep bandwidth $\Delta\lambda$ or $\Delta k$ is used for the intensity measurements, i.e. $\Delta\lambda_M = \Delta\lambda$, or $\Delta k_M = \Delta k$. The total of N measurements for intensity acquisition is then distributed to the entire spectral sweep bandwidth. In the case of equidistant sampling intervals, $\Delta\lambda = N\cdot\delta_{Sampling}\lambda$, or $\Delta k = N\cdot\delta_{Sampling}k$, then results. This is to be taken as a basis in the following, for which reason no distinction is made in the following between $\Delta\lambda_M$ and $\Delta\lambda$, or $\Delta k_M$ and $\Delta k$. In the following, therefore, the spectral bandwidth is to be understood to include both the spectral sweep bandwidth of the light source (i.e. the bandwidth by which the light source is tuned under the control of the control device) and the spectral measurement bandwidth. Note that all examples and embodiments in which $\Delta\lambda_M = \Delta\lambda$ or $\Delta k_M = \Delta k$ are not part of the claimed invention.

In a particular design, the control device can be switched over between a plurality of at least two predefined operating modes, which differ from one another in the measurement depth $z_{max}$ or/and in the axial (longitudinal) resolution $\Delta z$. By assignment to each of these operating modes, one or more parameter values necessary for setting the respective operating mode can then be stored in a memory that can be accessed by the control device. If a switchover is to be effected from one operating mode to another, the control device can read out from the memory the at least one parameter value stored for the new operating mode and, in accordance with the at least one parameter value that is read, can effect a corresponding adaptation of the operation of the OCT apparatus. The adaptation results in the values for the

measurement depth $z_{max}$ and for the axial resolution $\Delta z$, which are characteristic of the new operating mode.

The at least one parameter value that is stored can comprise, for example, the number N of intensity measurements to be performed per sweep. Alternatively or additionally, the at least one parameter value can comprise information that serves to specify the measurement bandwidth $\Delta\lambda_M$, $\Delta k_M$, for example a lower range limit or/and an upper range limit or/and the magnitude of the spectral sampling interval between two consecutive measurements. Alternatively or additionally, the at least one parameter value can comprise information that serves to specify the central wavelength $\lambda_0$ or the central wave number $k_0$, for example the central value, the median or the spectral centroid of the measurement bandwidth $\Delta\lambda_M$, $\Delta k_M$.

In a preferred embodiment, the measurement depth of the operating modes can in each case be matched to a differing length of portion of a human eye. Such an eye portion can comprise, for example, substantially only the human cornea over its entire thickness (i.e. from the anterior to the posterior corneal surface), or it can comprise, for example, the cornea up to and including the anterior chamber, but substantially excluding the human lens. Alternatively, it can comprise, for example, the cornea, the anterior chamber and the human lens, but without extending as far as the retina, or it can comprise, for example, all structures over the entire length from the cornea as far as the retina. From these example, it is clearly evident that a differing length of the imaged portions means an extent of differing length in the direction from the cornea as far as the retina, i.e. along the direction of the optical axis of the human eye. This is to be illustrated, in the following, in exemplary calculations:

(I) Imaging of cornea (high resolution)

| | |
|---|---|
| - measurement depth (desired): | $z_{max} \approx 3mm$ |
| - light source parameters: | $\lambda_0 = 1050nm$, $\Delta\lambda_{FW} = 100nm$ |
| - axial resolution according to (8): | $\Delta z_{rec} \approx 5.5\mu m$ (in air, n=1) |
| | $\Delta z_{rec} \approx 4.0\mu m$ (in the cornea, n≈1.38) |
| - required number of measurement points according to (12): | N ≈ 1090 (air) |
| | N ≈ 1500 (cornea) |

(II) Imaging of lens (high resolution)

| | |
|---|---|
| - measurement depth (desired): | $z_{max} \approx 4.5mm$ |
| - light source parameters: | as in (I) |
| - axial resolution according to (8): | $\Delta z_{rec} \approx 4.0\mu m$ (in the lens, n≈1.4) |
| - required number of measurement points according to (12): | N ≈ 2250 (lens) |

(III) Imaging of anterior chamber (high resolution)

| | |
|---|---|
| - measurement depth (desired): | $z_{max} \approx 4.5mm$ |
| - light source parameters: | as in (I) |
| - axial resolution according to (8): | $\Delta z_{rec} \approx 4.0\mu m$ (in the anterior chamber, n≈1.34) |
| - required number of measurement points according to (12): | N ≈ 2250 (anterior chamber) |

(IV) Anterior chamber and lens (in the case of a defined number of measurement points)

| | |
|---|---|
| - measurement depth (desired): | $z_{max} \approx 9mm$ |
| - light source parameter ($\lambda_0$ unaltered): | $\lambda_0 = 1050nm$ |
| - number of measurement points: | N ≈ 1500 |
| - axial resolution according to (8): | $\Delta z_{rec} \approx 12.0\mu m$ (in the anterior chamber, n≈1.34) |
| | $\Delta z_{rec} \approx 12.0\mu m$ (in the lens, n≈1.4) |
| - required $\Delta\lambda_{FW}$ of the light source according to (8): | $\Delta\lambda_{FW} \approx 46nm$ |

(V) Total eye length

| | |
|---|---|
| - measurement depth (desired): | $z_{max} \approx 25mm$ |
| - light source parameter ($\lambda_0$ unaltered): | $\lambda_0 = 1050nm$ |
| - axial resolution (desired): | $\Delta z_{rec} = 10.0\mu m$ |

(continued)

- required number of measurement points according to (12):  $N \approx 5000$ (for $n \approx 1$)
- required $\Delta\lambda_{FW}$ of the light source according to (8):  $\Delta\lambda_{FW} \approx 55nm$

Predefining, in such a manner, differing operating modes whose measurement depth is matched to eye portions of differing lengths makes it possible optionally to display, e.g. on a monitor screen, sections of an eye that differ in size. These sections can at the same time be combined with differing axial resolutions, such that, for example, an operating mode having a greater measurement depth has a more coarse or rough axial resolution than another operating mode that has a smaller measurement depth but, for that, has a finer axial resolution.

The apparatus can comprise a user interface arrangement, which is connected to the control device and allows a trigger signal to be input by a user. The control device can be set up to automatically compile a first tomogram of the object, in a first operating mode, upon input of the trigger signal, and then, in a second operating mode that differs from the first operating mode, to compile a second tomogram of the object.

The first and/or the second tomogram can constitute a one-, two- or three-dimensional tomogram. The object can comprise at least one element of the following group: a human eye, a part of a human eye, the cornea of an eye, the anterior chamber of an eye, the iris of an eye, the posterior chamber of an eye, the lens of an eye, the vitreous body of an eye, the retina of an eye, an applanation plate, a test plate and/or a pattern on and/or within a test plate. Accordingly, the object can also be understood as an object group consisting of at least two sub-objects. The test plate can be composed of polymethyl methacrylate (in short: PMMA).

A measurement depth of the first operating mode can be greater than the measurement depth of the second operating mode. Preferably, the measurement depth of the first operating mode is matched to a first portion of the object, and the measurement depth of the second operating mode is matched to a second portion of the object. The second portion can be a sub-portion of the first portion. Preferably, the axial resolution of the second operating mode is finer than the axial resolution of the first operating mode.

The control device can further be set up to identify in the first tomogram, by means of image processing, at least one fist feature of the object. The control device can further be set up to identify in the second tomogram, by means of image processing, at least one second feature of the object. In particular, the control device can be set up to identify in the second tomogram, by means of image processing, the at least one first feature of the object also identified in the first tomogram, and at least one second feature of the object. The control device can also be set up to ascertain the shape, position and/or orientation of the first feature in the first tomogram relative to the shape, position and/or orientation of the first and/or second feature in the second tomogram. The control device can also be set up to reference the first and the second tomogram to one another on the basis of the shapes, positions and/or orientations of the first and/or second feature.

In a preferred development, the OCT apparatus can comprise a user interface arrangement, which is connected to the control device and which allows a user to input instructions that effect an alteration of the measurement depth or/and of the axial resolution. This enables a user of the OCT apparatus to perform the tomography, in a manner elected by the user, with a differing measurement depth or/and differing axial resolution. In particular, the alteration of the measurement depth or/and of the axial resolution can be effected by the user in that the latter switches over between a plurality of predefined operating modes of the OCT apparatus that each differ from one another in their differing measurement depth or/and their differing axial resolution.

This does not preclude the user from being able to effect a continuous adjustment, e.g. of the measurement depth, via the user interface arrangement. It is also not precluded that the control device can effect automatic switchover from one of the predefined operating modes to another, for example controlled through corresponding instructions in a software program for an examination to be performed using the OCT apparatus.

The user interface arrangement can comprise, for example, a mechanical key arrangement or a key arrangement realized by means of a touch-pad (e.g. touch-screen), by means of which the user can call up the respectively desired operating mode. Alternatively or additionally, the user interface arrangement, for the purpose of switching over between differing operating modes, can comprise a button arrangement, which can be represented on a graphical user interface (in short: GUI) on a monitor screen and which can be controlled by the user with the aid of an electronic pointer device (e.g. mouse, trackball, keyboard).

For example, the user interface arrangement in this case can comprise appropriate visual instructions providing the user with information concerning which portion of the human eye the respectively selected operating mode corresponds to.

For the user, operation of the OCT apparatus is thus made particularly simple and clear.

The control device can be set up to control the light source and the detector in such a way that the detector performs the intensity acquisitions in accordance with a defined clock signal. The clock signal can be characterized by a fixed timing. In particular, the clock signal can be adapted in such a way that the detector performs the intensity acquisitions linearly over the wave length λ or over the wave number k of the light emitted by the light source. In the latter case, a Mach-Zehnder interferometer can be provided for determining the clock signal, a portion of the light emitted by the light source being coupled into the Mach-Zehnder interferometer, and the intensity of the auto-correlation signal being acquired as a function of time, on the basis of which a clock signal is determined, which clock signal is transmitted to the control device and forwarded by the latter to the detector.

Within the scope of the invention, the OCT apparatus can be realized as an autonomous device that serves, if necessary together with further diagnostic modules, purely for diagnostics. It is also conceivable, however, for the OCT apparatus to be integrated into a laser-assisted treatment system that can be used to perform laser-surgery interventions on a human eye, for instance to produce incisions in the eye tissue by photodisruption or to ablate corneal eye tissue. Such a treatment system typically comprises a laser source that provides pulsed laser radiation, for example in the UV or near-IR wavelength range, controllable scanning components for spatially setting a focal position of the laser radiation, and focussing optics for focussing the laser radiation. Insofar as a source other than the laser source of the treatment system is used for generating the emitted light of the OCT apparatus (measurement light), the same focussing optics as used for the laser radiation of the treatment system can nevertheless be used to focus the measurement light of the OCT apparatus onto the eye to be examined/treated.

We further describe a method for optical swept-source coherence tomography. The method comprises the steps of:

- emitting coherent light from a spectrally tuneable source,
- acquiring the intensity of interference light by means of a detector, the interference light resulting from superposing, on reference light, remitted light backscattered from an object irradiated with the coherent light of the source, and
- controlling the light source and the detector by means of a control device, in such a way that the detector performs intensity acquisitions in accordance with a defined number of measurements, while the light source is tuned, the defined number of measurements or/and a spectral measurement bandwidth, within which the detector performs the intensity acquisitions, being altered by means of the control device, for the purpose of altering the measurement depth or/and the axial resolution of the tomography.

Preferably, in the case of the method, the control device is used to switch over between a plurality of at least two predefined operating modes, which differ from one another in the measurement depth or/and in the axial resolution. One of the operating modes can have a finer axial resolution, but a shorter measurement depth, than another of the operating modes. The operating modes differ from one another in their differing measurement depths, the measurement depth of each operating mode being matched to a portion of the object, in particular of a human eye, of differing length. In the case of the method, upon input of a trigger signal by a user, a first tomogram of the object can be compiled automatically, in a first operating mode, and then, in a second operating mode that differs from the first operating mode, a second tomogram can be compiled.

In the case of the method, an alteration of the measurement depth or/and or the axial resolution can be effected through input of instructions by a user.

The detector is preferably controlled by means of the control device in such a way that the detector performs the intensity acquisitions in accordance with a defined clock signal.

The clock signal can be characterized by a fixed timing. The clock signal can be adapted in such a way that the detector performs the intensity acquisitions linearly over the wavelength of the light emitted by the light source. Alternatively, the clock signal can be adapted in such a way that the detector performs the intensity acquisitions linearly over the wave number of the light emitted by the light source.

The clock signal can further be determined by means of a Mach-Zehnder interferometer, which is connected to the control device and into which a portion of the light emitted by the light source is coupled for the purpose of auto-correlation, and which is set up to iteratively acquire the intensity of the auto-correlation signal as a function of time.

Insofar as a method, or individual steps of a method, for optical swept-source coherence tomography is/are described in this description, the method, or individual steps of the method, can be executed by a corresponding apparatus for optical swept-source coherence tomography, The same applies to the explanation of the manner of operation of an apparatus that executes the method steps. To that extent, the method feature and the apparatus features of this description are equivalent.

## Brief Description of the Drawings

The invention is explained further in the following with reference to the appended figures, wherein:

Fig. 1 shows a schematic block representation of an apparatus for optical swept-source coherence tomography, according to a first embodiment,

Fig. 2 shows a schematic block representation of an apparatus for optical swept-source coherence tomography, according to a second embodiment,

Fig. 3 shows a schematic block representation of an apparatus for optical swept-source coherence tomography, according to a third embodiment,

Fig. 4 shows a representation of a pattern made in a PMMA plate,

Figs. 5a and 5b show a representation of an applanation plate and of an eye in the non-flattened state and in the flattened state, and

Fig. 6 shows a representation of an eye, the iris and the pupil.

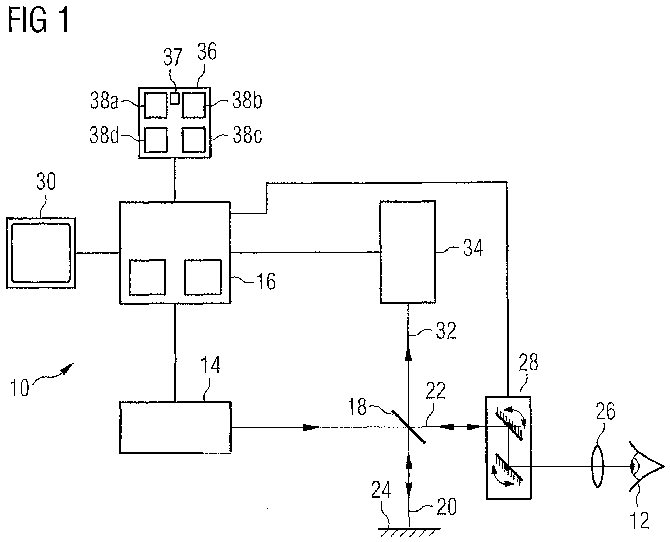

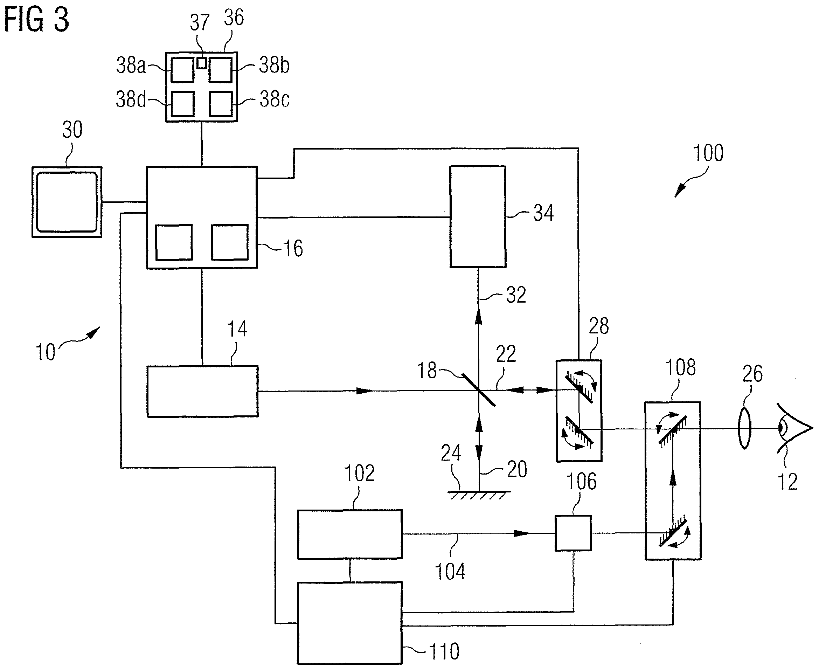

In Figs. 1 and 3, an apparatus for optical swept-source coherence topography is denoted in general by 10. In the example, the apparatus 10 is used to examine an object, shown as a human eye 12.

A first embodiment of the apparatus 10 is shown schematically in Fig. 1. The apparatus 10 comprises a spectrally tuneable light source 14 for emitting coherent light of a narrow, instantaneous line width. The light source 14 is realized as a swept-source light source and is tuned within the spectral sweep bandwidth $\Delta\lambda$ defined by a control device 16, in the wavelength $\lambda$ of the emitted light, in accordance with a tuning curve. The tuning curve, which describes the variation of the output wavelength as a function over time, has a linear, substantially liner or approximately linear, unidirectional tuning characteristic, from a short to a long wavelength $\lambda$, or a bidirectional tuning characteristic, from a short to a long to a short wavelength $\lambda$.

The tuning of the light source 14 is effected by varying the resonator length of the light source 14 realized as a laser. A so-called tuneable filter is used for this purpose. For example, a Fabry-Perot etalon disposed in the beam path within the resonator, or a grating that defines the resonator length of the laser in a Littrow configuration, is tilted relative to the direction of propagation of the laser beam in the resonator. Adaptation of the spectral range used, and therefore switchover of the resolution, is possible for tuneable filters operated in a resonant manner and tuneable filters operated in a non-resonant manner. MEMS-based SS light sources are mostly operated in a resonant manner, whereas piezo-based systems are not necessarily operated in a resonant manner. Alternatively, a polygon mirror can also be used.

The light emitted by the light source 14 is directed onto a beam splitter 18. The beam splitter 18 is a constituent part of an interferometer, and splits the incident optical power in accordance with a defined division ratio, for example 50:50. The one beam 20 runs within the reference arm, the other beam 22 running within the sampling arm.

The light branched off into the reference arm is incident on a mirror 24, which reflects the light back in a collinear manner onto the beam splitter 18. The distance in the reference arm between the mirror 24 and the beam splitter 18 is constant in respect of time. The light branched off into the sampling arm is incident on the object 12 to be examined, which scatters, or reflects, the light back in the direction of the beam splitter 18. Provided within the sampling arm are further optical elements 26 and positioning components 28, which are set up to focus the incoming light beam 22 from the beam splitter 18 onto the object 12 and to adjust the focal position in one of the two, or in both, lateral (i.e. transverse relative to the direction of beam propagation) directions. The control device 16 controls the positioning components 28 in a manner known per se, for the purpose of obtaining 2D or 3D tomograms. The computed tomograms are displayed on a display unit 30.

At the beam splitter 18, the light reflected back from the mirror 24 in the reference arm is superposed in a collinear manner on the light back-scattered from the object 12 in the sampling arm to form an interference beam 32. The optical path lengths in the reference arm and sampling arm are substantially equal, such that the interference beam 32 indicates an interference between the component beams 20, 22 from the reference arm and sampling arm. A detector that comprises a photodiode, or a balanced detector 34, acquires the intensity of the interference beam 32 as a function of time, in accordance with a number N of measurements defined by the control device 16. The number N of measurements/samplings corresponds to a number of trigger signals for acquisition electronics of the detector 34.

Through simultaneous tuning of the light source 14 and measuring of the intensity of the interference beam 32 by means of the detector 34, an interferogram is acquired over the wavelength $\lambda$, where the intensity measurements are distributed equidistantly over the wavelength $\lambda$.

For this purpose, the control device is set up to control the light source 14 and the detector 34 in such a way that the detector 34 performs the intensity acquisitions in accordance with a defined clock signal. The clock signal is adapted in such a way that the detector 34 performs the intensity acquisitions linearly over the wavelength $\lambda$ of the light emitted by the light source 14.

A user can provide user input using a user interface arrangement 36 connected to the control device 16 to switch back and forth between a plurality of operating modes, for example four operating modes. For this purpose, operating elements - denoted here by 38a, 38b, 38c, 38d - that can be actuated by the user are provided on the user interface arrangement 36. One of the operating elements 38a-d is assigned, respectively, to each operating mode in the example shown. Upon actuation of an operating element 38a-d, the control device 16 switches over to an operating mode assigned to the operating element 38a-d, and adapts the number of measurements and the acquired spectral bandwidth in such a way that the apparatus 10 acquires a tomogram that has a measurement depth $z_{max}$ assigned to the operating mode and has an axial resolution $\Delta z$ assigned to the operating mode.

Upon actuation of the first operating element 38a, the control device 16 switches, for example, into a first operating mode, and adapts the number of measurements and the acquired spectral bandwidth in such a way that the measurement depth $z_{max,1}$ is matched to a portion of the eye 12 that extends substantially along the optical axis of the eye 12, from the epithelial layer of the cornea as far as the retina, for the purpose of measuring the optical axial length of the eye 12, the axial resolution $\Delta z_1$ corresponding to a coarse or rough resolution.

Upon actuation of the second operating element 38b, the control device 16 switches, for example, into a second operating mode, and adapts the number of measurements and the acquired spectral bandwidth in such a way that the measurement depth $z_{max,2} < z_{max,1}$ is matched to a portion of the eye 12 that extends substantially along the optical axis of the eye 12, from the epithelial layer of the cornea as far as the boundary surface of the human lens that faces towards the retina, the axial resolution $\Delta z_2 < \Delta z_1$ corresponding to a less coarse or rough resolution.

Upon actuation of the third operating element 38c, the control device 16 switches, for example, into a third operating mode, and adapts the number of measurements and the acquired spectral bandwidth in such a way that the measurement depth $z_{max,3} < z_{max,2}$ is matched to a portion of the eye 12 that extends substantially along the optical axis of the eye 12, via the anterior eye chamber, the axial resolution $\Delta z_3 < \Delta z_2$ corresponding to a fine resolution.

Upon actuation of the fourth operating element 38d, the control device 16 switches, for example, into a fourth operating mode, and adapts the number of measurements and the acquired spectral bandwidth in such a way that the measurement depth $z_{max,4} < z_{max,3}$ is matched to a portion of the eye 12 that extends substantially along the optical axis of the eye 12, from the epithelial layer as far as the endothelial layer of the cornea, the axial resolution $\Delta z_4 < \Delta z_3$ corresponding to a very fine resolution,

Apparatus 10 may have any suitable number of operating modes, such as two, three, four, or more operating modes.

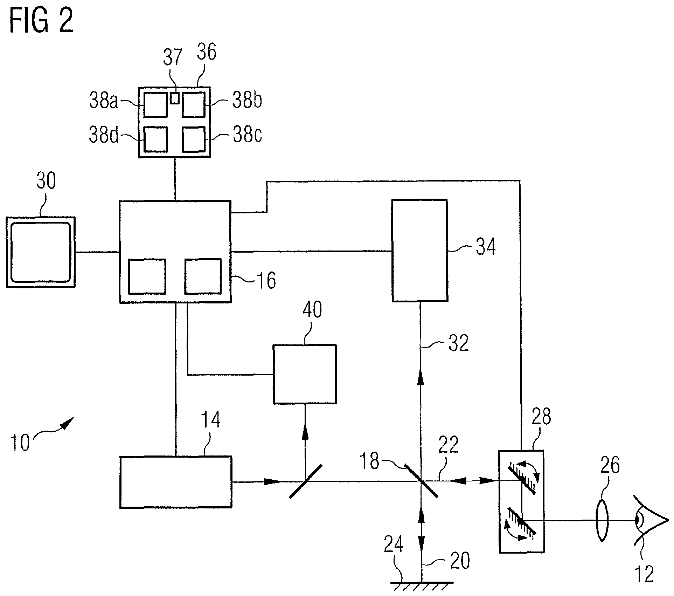

A second embodiment of the apparatus 10 is shown schematically in Fig. 2. In this figure, components that are the same or perform the same function as those in Fig. 1 are denoted by the same references. However, the SS light source 14 in Fig. 2 is tuned in such a way that the tuning curve is approximately linearly and the detection is non linearly in λ, i.e. not chronologically equidistant. In this case, both a unidirectional and a bidirectional tuning characteristic of the wave length λ as a function of time may be used. Through simultaneous tuning of the light source 14 and measuring of the intensity of the interference beam 32 by means of the detector 34, an interferogram is acquired over the wave number k, where the intensity measurements are distributed equidistantly over the wave number k.

For this purpose, the control device 16 is set up to control the light source 14 and the detector 34 in such a way that the detector 34 performs the intensity acquisitions in accordance with a defined clock signal. The clock signal is adapted in such a way that the detector 34 performs the intensity acquisitions linearly over the wave number k of the light emitted by the light source 14. For the purpose of determining the clock signal, a Mach-Zehnder interferometer 40 is provided, into which a portion of the light emitted by the light source 14 is coupled for the purpose of auto-correlation. The Mach-Zehnder interferometer 40 acquires the intensity of the auto-correlation signal as a function of time and, on the basis of that, determines a linear clock signal for change in the wave number k. The clock signal is transmitted to the control device 16 and forwarded by the latter for timing the detector 34.

The Mach-Zehnder interferometer 40 is therefore used by the apparatus 10 as a linear-k clock. The linear-k clock generates trigger signals. Upon each trigger signal, intensity is measured. The number of trigger signals of the linear-k clock in this case is dependent on the total spectral width of the light source 14 and, in particular, on the arm length difference $\Delta z_{MZI}$ of the Mach-Zehnder interferometer 40. The arm length of the Mach-Zehnder interferometer 40 is adapted in the case of a change in the spectral range in order to continue to obtain a constant number of sampling signals in accordance with

$$N = 2\frac{\Delta\lambda}{\lambda_0^2}\Delta z_{MZI} \qquad (15)$$

Fig. 3 shows a third embodiment of the apparatus 10. In this figure, components that are the same or perform the same function as those in Figs. 1 and 2 are denoted by the same references. The apparatus 10 according to Fig. 3 is part of a treatment system 100 for ophthalmological laser surgery. In this case, the apparatus 10 is used to examine the eye 12, and the treatment system 100 is used for ophthalmological treatment of the eye 12.

The treatment system 100 comprises a laser source 102 for providing laser radiation 104, controllable scanning components 106 for setting a focal position of the laser radiation 104 in the axial direction, and controllable scanning components 108 for setting the focal position of the laser radiation 104 in the axial direction. The laser radiation 104 is focussed onto or into the eye 12 by means of the optical element 26. A control arrangement 110 that controls the laser source 102 and the scanning components 106, 108 causes the scanning components 106 or/and 108 to effect a scanning movement, such that the focal position of the laser radiation 104 at or in the eye 12 follows a defined path. The laser radiation 104 acts upon the tissue of the eye 12 by photodisruption, for example for the purpose of refractive correction. Further, for persons skilled in the art, it is understood that the treatment system 100 can also be used for other applications in the 2D or 3D field such as, for example, keratoplasty, in particular DALK (deep anterior lamellar keratoplasty).

The control device 16 of the apparatus 10 is connected to the control arrangement 110 of the treatment system 100, and transmits to the latter control arrangement structure data of the eye 12, which data is based on the tomogram acquired by the apparatus 10. The control arrangement 110 adapts the path of the focal position of the laser radiation 104 on the basis of the structure data in such a way that a optimal ophthalmological result can be achieved.

The structure for beam guidance shown in Figs. 1 to 3, which is not specified in greater detail, can be wholly or partially supplemented or replaced by fibre-based optics components. For example, the Mach-Zehnder interferometer 40 shown in Fig. 2 can have a fibre-based structure.

The user interface arrangement 36 further comprises a trigger device 37, which allows a trigger signal to be input by a user. The control device 16 is set up to automatically generate a first tomogram of the object 12, in a first operating mode, upon input of the trigger signal, and then, in a second operating mode that differs from the first operating mode, to generate a second tomogram of the object 12.

In the context of a first exemplary application, the object 12 is constituted by a PMMA plate 12', which, prior to the treatment of a patient's eye, is disposed in the region of the eye 12 represented in Figs. 1 to 3 in such a way that it has a fixed relative position and orientation in relation to the optical element 26 and consequently, to the entire apparatus 10. The PMMA plate 12' is used for functional testing and calibration of the treatment system 100. The PMMA plate 12' is composed of PMMA plastic. The control arrangement 110, by correspondingly controlling the laser source 102 and the scanning components 106, 108, effects a pattern 50 made on and/or in the PMMA plate 12'. The pattern 50 is produced by the laser radiation 104 acting upon the PMMA plastic.

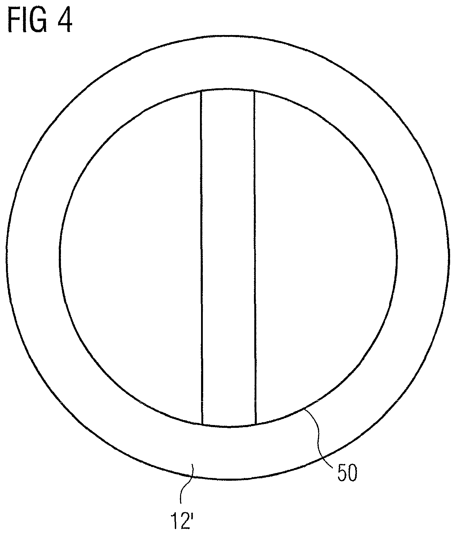

Fig. 4 shows a top view of the PMMA plate 12' and the effected pattern 50. The PMMA plate 12' has, for example, a cylindrical geometry. In the example shown here, the pattern 50 comprises a circle having two inscribed straight lines running parallelwise. In a first operating mode, upon input of the trigger signal the control device 16 automatically compiles a first tomogram, from the pattern 50. The measurement depth of the first operating mode in this case is matched to a length that represents the geometry of the pattern 50. Then, in a second operating mode, the control device 16 automatically compiles a second tomogram, from the entire PMMA plate 12', including the pattern 50 effected therein/thereon. The measurement depth of the second operating mode in this case is matched to a length that represents the geometry of the PMMA plate 12'. The resolution of the first tomogram is finer than the resolution of the second tomogram.

By means of image processing, the control device 16 identifies, in the first tomogram, the circle and the two straight lines of the pattern 50 that run parallelwise. In addition, by means of image processing, the control device identifies, in the second tomogram, the circle and the two straight lines of the pattern 50 that run parallelwise, as well as the two base surfaces of the PMMA plate 12'. The control device 16 determines therefrom the positions and orientations of the circle and of the two straight lines of the pattern 50 that run parallelwise relative to the positions and the orientations of the circle and of the two straight lines of the pattern 50 that run parallelwise, as well as of the two base surfaces of the PMMA plate 12'. This enables the first tomogram to be referenced to the second tomogram. In particular, this enables the pattern 50, or parts thereof, to be referenced in relation to the PMMA plate 12', the apparatus 10 or the treatment system 100.

In the context of a second exemplary application, the object 12 is constituted by an applanation plate 12", which, in the treatment of a patient's eye 12, constitutes the contact element between the eye 12 and the apparatus 10, or treatment system 100, and which is used to flatten the cornea 42 of the eye 12 on to the apparatus 10, or treatment system 100. For example, the applanation plate 12" is disposed between the eye 12 represented in Figs. 1 to 3 and the optical element 26.

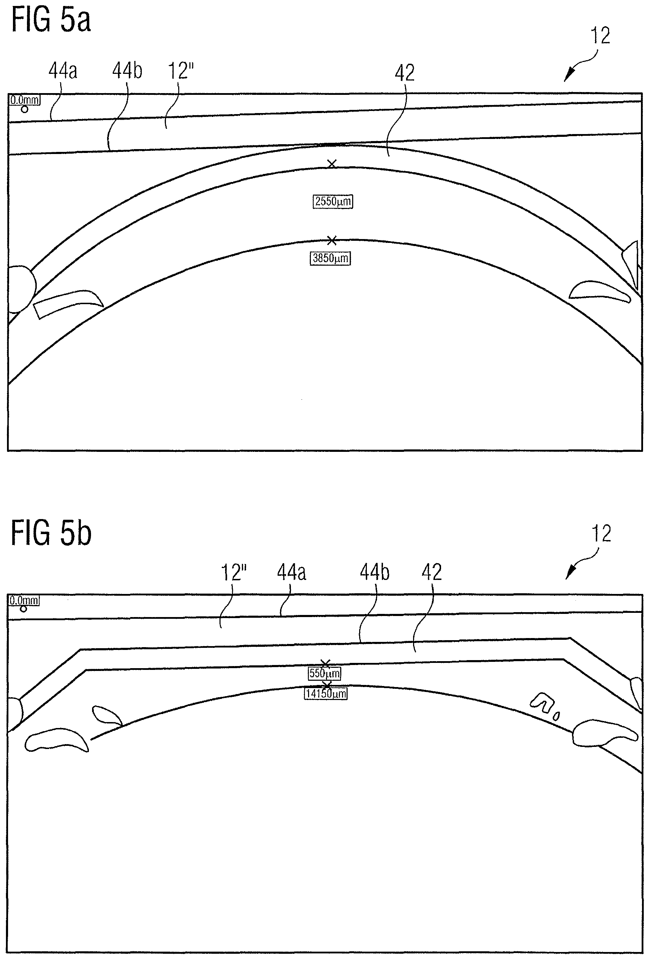

In Fig. 5a, the eye can be seen in an as yet non-flattened state, while in Fig. 5b the cornea 42 of the eye 12 has already been flattened. In a first operating mode, upon input of the trigger signal the control device 16 automatically compiles a first tomogram, from the applanation plate 12". The measurement depth of the first operating mode in this case is matched to a length that represents the geometry of the applanation plate 12". Then, in a second operating mode, the control device 16 automatically compiles a second tomogram, from a system comprising the eye 12, in the flattened state, and the applanation plate 12".

The measurement depth of the second operating mode in this case is matched to a length that represents the geometry of the system consisting of the eye 12 and the applanation plate 12". The resolution of the first tomogram is finer than the resolution of the second tomogram.

By means of image processing, the control device 16 identifies the two base surfaces 44a, 44b of the applanation plate 12" in the first tomogram. In addition, by means of image processing, the control device identifies the two base surfaces 44a, 44b of the applanation plate 12" in the second tomogram. The control device determines therefrom the positions and orientations of the two base surfaces of the applanation plate 12" in the first tomogram relative to the positions and orientations of the two base surfaces of the applanation plate 12" in the second tomogram. This makes it possible to determine the position and the position tolerances of the applanation plate 12" and to reference the eye 12 in relation to the position of the applanation plate 12".

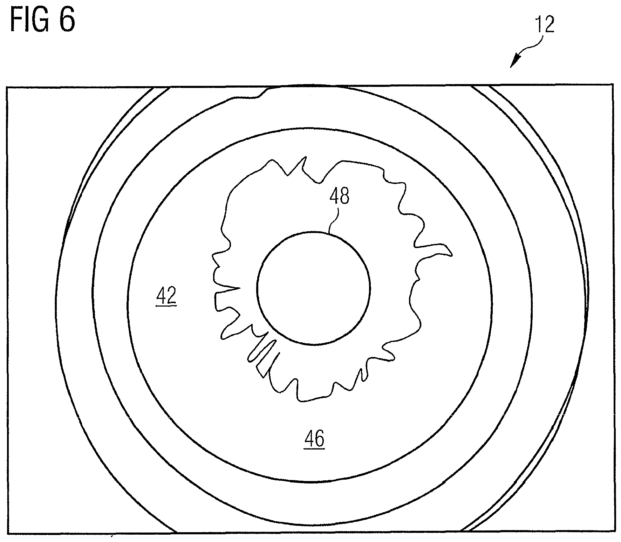

In the context of a third exemplary application that relates to keratoplasty, the object 12 is again constituted by a patient's eye 12. Fig. 6 shows the eye 12, and the cornea 42, the iris 46 and the pupil 48 of the eye 12. In a first operating mode, upon input of the trigger signal the control device 16 automatically compiles a first tomogram, from the cornea 42 of the eye 12. The measurement depth of the first operating mode in this case is matched to a length that represents the geometry of the cornea 42. Then, in a second operating mode, the control device 16 automatically compiles a second tomogram, from the system consisting of the cornea 42, the anterior chamber and the iris 46 of the eye 12. The measurement depth of the second operating mode in this case is matched to a length that represents the geometry of the system consisting of the cornea 42, the anterior chamber and the iris 46. The resolution of the first tomogram is finer than the resolution of the second tomogram.

By means of image processing, the control device 16 identifies, in the first tomogram, the shape, position and orientation of the cornea 42. In addition, by means of image processing, the control device 16 identifies, in the second tomogram, the shape, position and orientation of the cornea 42, the iris 46 and the pupil 48. The control device determines therefrom the shape, position and orientation of the cornea 42 in the first tomogram relative to the shape, position and orientation of the cornea 42, the iris 46 and the pupil 48 in the second tomogram. This enables the cornea 42 to be referenced in relation to the iris 46 and/or pupil 48.

In the context of a fourth exemplary application that relates to cataract treatment, the object 12 is again constituted by a patient's eye 12. In a first operating mode, upon input of the trigger signal the control device 16 automatically compiles a first tomogram, from the human lens of the eye 12. The measurement depth of the first operating mode in this case is matched to a length that represents the geometry of the lens. Then, in a second operating mode, the control device 16 automatically compiles a second tomogram, from the entire eye 12. The measurement depth of the second operating mode in this case is matched to a length that represents the geometry of the entire eye 12. The resolution of the first tomogram is finer than the resolution of the second tomogram.

By means of image processing, the control device 16 identifies, in the first tomogram, the shape, position and orientation of the lens. In addition, by means of image processing, the control device 16 identifies, in the second tomogram, the shapes, positions and orientations of the cornea, the lens and the retina of the eye 12. The control device 16 determines therefrom the shape, position and orientation of the lens in the first tomogram relative to the shapes, positions and orientations of the cornea, the lens and the retina in the second tomogram. This enables the lens to be referenced in relation to the optical axis of the eye 12.

**Claims**

1.   An apparatus (10) for optical swept-source coherence tomography, comprising:

   a spectrally tuneable source (14) for emitting coherent light;
   a detector (34) for acquiring the intensity of interference light that results from superposing, on reference light, remitted light backscattered from an object (12) irradiated with the coherent light of the source, and
   a control device (16) for controlling the light source (14) and the detector (34) in such a way that the detector (34) performs intensity acquisitions in accordance with a defined number of measurements, while the light source (14) is tuned;
   wherein the control device is configured to alter a spectral measurement bandwidth within which the detector (34) performs the intensity acquisitions while simultaneously keeping constant the number of measurements to alter the axial resolution,
   wherein the measurement bandwidth is less than a sweep bandwidth of the tuned light source (14).

2.   The apparatus (10) of Claim 1, wherein the control device (16) is configured to switch between a plurality of at least two predefined operating modes, which differ from one another in the measurement depth and the axial resolution.

3.   The apparatus (10) of Claim 2, wherein one of the operating modes has a finer axial resolution and a shorter measurement depth than another of the operating modes.

4.   The apparatus (10) of any one of Claims 1 to 3, wherein the control device (16) is configured to control the detector (34) in such a way that the detector (34) performs the intensity acquisitions in accordance with a clock signal.

5. The apparatus (10) of Claim 4, wherein the clock signal has a periodic timing.

6. The apparatus (10) of Claim 4 or 5, wherein:

- the clock signal has a timing adapted to allow the detector (34) to perform the intensity acquisitions linearly over the wavelength of the light emitted by the light source (14), or
- the clock signal has a timing adapted to allow the detector (34) to perform the intensity acquisitions linearly over the wave number of the light emitted by the light source (14), the apparatus (10) comprising a Mach-Zehnder interferometer connected to the control device and arranged to receive a portion of the light emitted by the light source for determining an auto-correlation signal of the received light, wherein the Mach-Zehnder interferometer is adapted to generate the clock signal by iteratively acquiring the intensity of the auto-correlation signal as a function of time.

7. A method for optical swept-source coherence tomography, comprising:

emitting coherent light from a spectrally tuneable source (14),
acquiring the intensity of interference light by means of a detector (34), the interference light resulting from superposing, on reference light, remitted light backscattered from an object (12) irradiated with the coherent light of the source; and
controlling the light source (14) and the detector (34) in such a way that the detector (34) performs intensity acquisitions in accordance with a defined number of measurements, while the light source (14) is tuned;
wherein a spectral measurement bandwidth, within which the detector (34) performs the intensity acquisitions, is altered while simultaneously keeping constant the number of measurements for altering an axial resolution of the tomography,
wherein the measurement bandwidth is less than a sweep bandwidth of the tuned light source (14).

8. The method of Claim 7, comprising:
switching between a plurality of at least two predefined operating modes, which differ from one another in the measurement depth and the axial resolution.

9. The method of Claim 8, wherein one of the operating modes has a finer axial resolution and a shorter measurement depth than another of the operating modes

10. The method of any of Claims 7 to 9, comprising:

generating a clock signal; and
controlling the detector (34) to perform the intensity acquisitions in accordance with the clock signal.

11. The method of Claim 10, wherein the clock signal has a periodic timing.

12. The method of Claim 10 or 11, wherein:

- the clock signal has a timing adapted to allow the detector (34) to perform the intensity acquisitions linearly over the wavelength of the emitted light (14), or
- the clock signal has a timing adapted to allow the detector (34) to perform the intensity acquisitions linearly over the wave number of the emitted light (14), the method comprising directing a portion of the emitted light to a Mach-Zehnder interferometer to generate an auto-correlation signal of the directed light and determining the clock signal based on the auto-correlation signal.


**Patentansprüche**

1. Vorrichtung (10) für eine optische durchstimmbare (Swept-Source) Kohärenztomografie, umfassend:

eine spektral abstimmbare Quelle (14) zum Ausstrahlen eines kohärenten Lichts;
einen Detektor (34) zum Erfassen der Intensität eines Interferenzlichts, das aus einer Überlagerung eines remittierten Lichts, das von einem Objekt (12) zurückgestreut wird, das mit dem kohärenten Licht der Quelle bestrahlt wird, auf ein Referenzlicht resultiert, und

eine Steuervorrichtung (16) zum Steuern der Lichtquelle (14) und des Detektors (34), sodass der Detektor (34) Intensitätserfassungen gemäß einer definierten Anzahl von Messungen ausführt, während die Lichtquelle (14) abgestimmt wird;

wobei die Steuervorrichtung konfiguriert ist zum Verändern einer spektralen Messbandbreite, innerhalb welcher der Detektor (34) die Intensitätserfassungen ausführt, während gleichzeitig die Anzahl der Messungen konstant gehalten wird, um die axiale Auflösung zu verändern,

wobei die Messbandbreite geringer als eine Durchlaufbandbreite der abgestimmten Lichtquelle (14) ist.

2. Vorrichtung (10) nach Anspruch 1, wobei die Steuervorrichtung (16) konfiguriert ist zum Umschalten zwischen einer Vielzahl von mindestens zwei vordefinierten Betriebsmodi, die sich durch die Messtiefe und die axiale Auflösung voneinander unterscheiden.

3. Vorrichtung (10) nach Anspruch 2, wobei einer der Betriebsmodi eine feinere axiale Auflösung und eine kürzerer Messtiefe als ein anderer der Betriebsmodi aufweist.

4. Vorrichtung (10) nach einem der Ansprüche 1 bis 3, wobei die Steuervorrichtung (16) konfiguriert ist zum Steuern des Detektors (34), sodass der Detektor (34) die Intensitätserfassungen gemäß einem Taktsignal ausführt.

5. Vorrichtung (10) nach Anspruch 4, wobei das Taktsignal einen regelmäßigen Zeitablauf aufweist.

6. Vorrichtung (10) nach Anspruch 4 oder 5, wobei:

- das Taktsignal einen Zeitablauf aufweist, der geeignet ist zum Erlauben, dass der Detektor (34) die Intensitätserfassungen linear über die Wellenlänge des Lichts ausführt, das von der Lichtquelle (14) abgestrahlt wird, oder

- das Taktsignal einen Zeitablauf aufweist, der geeignet ist zum Erlauben, dass der Detektor (34) die Intensitätserfassungen linear über die Wellenzahl des Lichts ausführt, das von der Lichtquelle (14) abgestrahlt wird, wobei die Vorrichtung (10) ein Mach-Zehnder-Interferometer umfasst, das mit der Steuervorrichtung verbunden ist und geeignet ist zum Empfangen eines Teils des Lichts, das von der Lichtquelle abgestrahlt wird, um ein Autokorrelationssignal des empfangenen Lichts zu ermitteln, wobei das Mach-Zehnder-Interferometer geeignet ist zum Erzeugen des Taktsignals, indem die Intensität des Autokorrelationssignals iterativ als eine Funktion der Zeit erfasst wird.

7. Verfahren für eine optische durchstimmbare (Swept-Source) Kohärenztomografie, umfassend:

Abstrahlen eines kohärenten Lichts von einer spektral abstimmbaren Quelle (14),

Erfassen der Intensität eines Interferenzlichts mithilfe eines Detektors (34), wobei das Interferenzlicht aus einer Überlagerung eines remittierten Lichts, das von einem Objekt (12) zurückgestreut wird, das mit dem kohärenten Licht der Quelle bestrahlt wird, auf ein Referenzlicht resultiert; und

Steuern der Lichtquelle (14) und des Detektors (34), sodass der Detektor (34) Intensitätserfassungen gemäß einer definierten Anzahl von Messungen ausführt, während die Lichtquelle (14) abgestimmt wird;

wobei eine spektrale Messbandbreite, innerhalb welcher der Detektor (34) die Intensitätserfassungen ausführt, verändert wird, während gleichzeitig die Anzahl der Messungen konstant gehalten wird, um die axiale Auflösung der Tomografie zu verändern,

wobei die Messbandbreite geringer als eine Durchlaufbandbreite der abgestimmten Lichtquelle (14) ist.

8. Verfahren nach Anspruch 7, umfassend:
Umschalten zwischen einer Vielzahl von mindestens zwei vordefinierten Betriebsmodi, die sich durch die Messtiefe und die axiale Auflösung voneinander unterscheiden.

9. Verfahren nach Anspruch 8, wobei einer der Betriebsmodi eine feinere axiale Auflösung und eine kürzerer Messtiefe als ein anderer der Betriebsmodi aufweist.

10. Verfahren nach einem der Ansprüche 7 bis 9, umfassend:

Erzeugen eines Taktsignals; und

Steuern des Detektors (34), um die Intensitätserfassungen gemäß dem Taktsignal auszuführen.

**11.** Verfahren nach Anspruch 10, wobei das Taktsignal einen regelmäßigen Zeitablauf aufweist.

**12.** Verfahren nach Anspruch 10 oder 11, wobei:

- das Taktsignal einen Zeitablauf aufweist, der geeignet ist zum Erlauben, dass der Detektor (34) die Intensitätserfassungen linear über die Wellenlänge des abgestrahlten Lichts (14) ausführt, oder
- das Taktsignal einen Zeitablauf aufweist, der geeignet ist zum Erlauben, dass der Detektor (34) die Intensitätserfassungen linear über die Wellenzahl des abgestrahlten Lichts (14) ausführt, wobei das Verfahren ein Leiten eines Teils des abgestrahlten Lichts zu einem Mach-Zehnder-Interferometer umfasst, um ein Autokorrelationssignal des geleiteten Lichts zu erzeugen und um das Taktsignal aufgrund des Autokorrelationssignals zu ermitteln.

## Revendications

**1.** Appareil (10) pour tomographie à cohérence de source balayée optique, comprenant :

une source spectralement accordable (14) pour émettre une lumière cohérente ;
un détecteur (34) pour acquérir l'intensité de lumière d'interférence qui résulte de la superposition, sur une lumière de référence, de la lumière renvoyée par rétrodiffusion à partir d'un objet (12) irradié avec la lumière cohérente de la source, et
un dispositif de commande (16) pour commander la source de lumière (14) et le détecteur (34) de manière telle que le détecteur (34) effectue des acquisitions d'intensité en conformité avec un nombre défini de mesures, pendant que la source de lumière (14) est accordée ;
dans lequel le dispositif de commande est configuré pour modifier une largeur de bande de mesure spectrale au sein de laquelle le détecteur (34) effectue les acquisitions d'intensité tout en conservant simultanément une constance du nombre de mesures afin de modifier la résolution axiale,
dans lequel la largeur de bande de mesure est plus petite qu'une largeur de bande de balayage de la source de lumière accordée (14).

**2.** Appareil (10) de la revendication 1, dans lequel le dispositif de commande (16) est configuré pour commuter entre une pluralité d'au moins deux modes d'exploitation prédéfinis, qui diffèrent l'un de l'autre en termes de profondeur de mesure et de résolution axiale.

**3.** Appareil (10) de la revendication 2, dans lequel un des modes d'exploitation a une résolution axiale plus fine et une profondeur de mesure plus courte qu'un autre des modes d'exploitation.

**4.** Appareil (10) de n'importe laquelle des revendications 1 à 3, dans lequel le dispositif de commande (16) est configuré pour commander le détecteur (34) de manière telle que le détecteur (34) effectue les acquisitions d'intensité en conformité avec un signal d'horloge.

**5.** Appareil (10) de la revendication 4, dans lequel le signal d'horloge a un cadencement périodique.

**6.** Appareil (10) de la revendication 4 ou 5, dans lequel :

- le signal d'horloge a un cadencement conçu pour permettre au détecteur (34) d'effectuer les acquisitions d'intensité de manière linéaire sur la longueur d'onde de la lumière émise par la source de lumière (14), ou
- le signal d'horloge a un cadencement conçu pour permettre au détecteur (34) d'effectuer les acquisitions d'intensité de manière linéaire sur le nombre d'ondes de la lumière émise par la source de lumière (14), l'appareil (10) comprenant un interféromètre Mach-Zehnder qui est connecté au dispositif de commande et agencé pour recevoir une portion de la lumière émise par la source de lumière pour déterminer un signal d'auto-corrélation de la lumière reçue, l'interféromètre Mach-Zehnder étant conçu pour générer le signal d'horloge grâce à l'acquisition de manière itérative de l'intensité du signal d'auto-corrélation en tant que fonction du temps.

**7.** Procédé pour tomographie à cohérence de source balayée optique, comprenant :

l'émission d'une lumière cohérente à partir d'une source spectralement accordable (14),

l'acquisition de l'intensité de lumière d'interférence au moyen d'un détecteur (34), la lumière d'interférence résultant de la superposition, sur une lumière de référence, de la lumière renvoyée par rétrodiffusion à partir d'un objet (12) irradié avec la lumière cohérente de la source ; et

la commande de la source de lumière (14) et du détecteur (34) de manière telle que le détecteur (34) effectue des acquisitions d'intensité en conformité avec un nombre défini de mesures, pendant que la source de lumière (14) est accordée ;

dans lequel une largeur de bande de mesure spectrale, au sein de laquelle le détecteur (34) effectue les acquisitions d'intensité, est modifiée tout en conservant simultanément une constance du nombre de mesures afin de modifier une résolution axiale de la tomographie,

dans lequel la largeur de bande de mesure est plus petite qu'une largeur de bande de balayage de la source de lumière accordée (14).

8. Procédé de la revendication 7, comprenant :
la commutation entre une pluralité d'au moins deux modes d'exploitation prédéfinis, qui diffèrent l'un de l'autre en termes de profondeur de mesure et de résolution axiale.

9. Procédé de la revendication 8, dans lequel un des modes d'exploitation a une résolution axiale plus fine et une profondeur de mesure plus courte qu'un autre des modes d'exploitation.

10. Procédé de n'importe lesquelles des revendications 7 à 9, comprenant :

la génération d'un signal d'horloge ; et
la commande du détecteur (34) afin d'effectuer les acquisitions d'intensité en conformité avec le signal d'horloge.

11. Procédé de la revendication 10, dans lequel le signal d'horloge a un cadencement périodique.

12. Procédé de la revendication 10 ou 11, dans lequel :

- le signal d'horloge a un cadencement conçu pour permettre au détecteur (34) d'effectuer les acquisitions d'intensité de manière linéaire sur la longueur d'onde de la lumière émise (14), ou
- le signal d'horloge a un cadencement conçu pour permettre au détecteur (34) d'effectuer les acquisitions d'intensité de manière linéaire sur le nombre d'ondes de la lumière émise (14), le procédé comprenant le fait de diriger une portion de la lumière émise à un interféromètre Mach-Zehnder afin de générer un signal d'auto-corrélation de la lumière dirigée et de déterminer le signal d'horloge sur la base du signal d'auto-corrélation.

# FIG 1

FIG 2

FIG 3

EP 2 795 241 B1

FIG 4

## FIG 5a

## FIG 5b

FIG 6

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20110176142 A1

**Non-patent literature cited in the description**

- **M. GORA.** *Optics Express 14880,* 17 August 2009, vol. 17 (17
- **G. Y. LIU.** *Optics Express 14095,* 01 September 2008, vol. 16 (18
- **J. XI.** *Optics Express 9511,* 26 April 2010, vol. 18 (9
- **CHOMA et al.** Sensitivity advantage of swept source and Fourier domain optical coherence tomography. *Optics Express,* 2003, vol. 11, 2183-2189
- Optical Coherence Tomography - Technology and Applications. Springer-Verlag, 2008

- **JIEFENG XI et al.** Generic real-time uniform K-space sampling method for high-speed swept-Source optical coherence tomography. *Optics Express,* 26 April 2010, vol. 18 (9), 9511-9517
- **MICHALINA GORA et al.** Ultra high-speed swept source OCT imaging of the anterior segment of human eye at 200 kHz with adjustable imaging range. *Optics Express,* 17 August 2009, vol. 17 (17), 14880-14894